# EUROPÄISCHE PATENTSCHRIFT

(11) **EP 0 792 654 B1**
(45) Veröffentlichungstag und Bekanntmachung des Hinweises auf die Patenterteilung: **17.10.2001**
(21) Anmeldenummer: 97102287.6
(22) Anmeldetag: 13.02.1997
(51) Int. Cl.: A61L 29/00

(54) **Antibakterielle Wirkung aufweisender Körper zur Verwendung als medizinisches oder chirurgisches Hilfsmittel**
Articles with antibacterial activity for use as medical or surgical aids
Acticles à activité antibactérielle et leur utilisation en médecine et chirurgie

(30) Priorität: 27.02.1996 DE 19607314
(43) Veröffentlichungstag der Anmeldung: 03.09.1997
(73) Patentinhaber: Gentamed AG, 6442 Gersau (CH)
(72) Erfinder: Bähr, Judith M. geb. Green, 76530 Baden-Baden (DE)
(74) Vertreter: Quermann, Helmut, Dipl.-Ing.

(56) Entgegenhaltungen:
- EP-A- 0 247 590
- EP-A- 0 693 300
- DE-A- 4 316 920
- DE-A- 4 344 306
- US-A- 4 054 139
- US-A- 4 915 694

## Beschreibung

Die Erfindung betrifft einen antibakterielle Wirkung aufweisenden Körper aus Kunststoff zur Verwendung als medizinisches oder chirurgisches Hilfsmittel.

Es ist bekannt, daß Antibiotika, insbesondere Gentamycin, aus Kunststoffen auf Basis von Polymethacrylaten und/oder Polyacrylaten protrahiert freigegeben werden. Dabei folgt einem steilen initialen Konzentrationsabfall als Ausdruck der Freisetzung aus den äußersten Schichten des Kunststoffs eine fast konstante, über lange Zeiträume langsam abnehmende Freisetzung. Diese antibiotikahaltigen Kunststoffe sind beispielsweise als Knochenzement zum Befestigen von Endoprothesen, zum Beispiel Hüfttotalendoprothesen, oder bei der Auswechselung infizierter Endoprothesen verwendet worden. Zur Infektionsbekämpfung bzw. - prophylaxe wurden hierbei die Antibiotika vor der Auspolymerisation des Kunststoffes zugefügt. Aus der DE-PS 23 20 373 ist ferner ein antibiotikahaltiges Mittel in Kugelform bekannt, welches einen Kunststoff enthält, wobei das Antibiotikum in gleichförmiger Verteilung im Kunststoff vorliegt. Die Kugeln besitzen einen Durchmesser von 1 bis 20 mm und sind vorzugsweise mit Hilfe von Fäden oder Drähten miteinander verbunden. Dieses Mittel dient beispielsweise zur Ausfüllung von osteomyelitischen Höhlen zur Schaffung eines aseptischen Transplantatlagers usw.. Aus der EP 0 236 468 B1 ist ferner ein Stift zur Einführung in ein in einem Knochen befindliches Bohrloch, welcher aus einem Träger aus Kunststoff auf der Basis von Polymethacrylat und/oder Polyacrylat oder ähnlichen Materialien besteht, bekannt, der in gleichmäßiger Verteilung das Antibiotikum, vorzugsweise Gentamycin aufweist, wobei der Träger zylinderförmig ausgebildet ist und dessen Innenraum zur Aufnahme eines Knochennagels oder einer Knochenschraube eines Fixateur externe dient.

Aus der DE 36 27 487 A1 ist ferner eine Gefäßprothese in Schlauchform bekannt, wobei der Schlauch im wesentlichen aus Kunststoff besteht und dessen zu vernähende bzw. zu verklemmende Endbereiche zur Verringerung der Infektionsgefahr als Speicher für Antibiotikum ausgebildet sind. Der Schlauch wird mit den jeweils anzuschließenden Blutgefäßen vernäht und ist damit beispielsweise als Bypass wirksam. Eine ähnliche Gefäßprothese ist aus der US 3 425 814 bekannt. Dort weist die Innenwandung des Schlauches ein Antibiotikum auf.

Aus der EP 0 693 300 A2 ist ein Katheter mit mehreren nebeneinander angeordneten und sich über eine kurze Länge erstreckenden Längsschlitzen bekannt. Bei einer Verstopfung des distalen Austrittsendes des Katheters mittels eines Pfropfens oder dergleichen, tritt durch die Druckverformung des Katheters im Bereich der Längsschlitze die Infusionsflüssigkeit durch diese aus und es wird durch die Verformung des distalen Endes des Katheters der Pfropfen gelöst, womit sich die Längsschlitze wieder schließen.

Bei allen chirgurgischen Situationen, bei denen die Haut durchbohrt, eingeschnitten oder in anderer Weise verletzt wird, ist das Risiko, daß Mikroorganismen in das Körpergewebe eindringen und eine lokale oder systemische Infektion verursachen weit verbreitet. Die Wunde, die über einen längeren Zeitraum existiert, zum Beispiel infolge postoperativer Vakuumdrainagen, externer Fixiernägel in der Orthopädie usw. stellt ein erhebliches Infektionsrisiko für die Patienten dar.

Es ist Aufgabe der vorliegenden Erfindung, einen antibakterielle Wirkung aufweisenden Körper aus Kunststoff zur Verwendung als medizinisches oder chirurgisches Hilfsmittel so weiter zu bilden, daß er bei einfachster Gestaltung eine wirksame Prophylaxe betreffend Infektionen bzw. eine lokale Behandlung bereits existierender Infektionen im Wundbereich ermöglicht.

Gelöst wird die Aufgabe durch einen antibakterielle Wirkung aufweisenden Körper zur Verwendung als medizinisches oder chirurgisches Hilfsmittel, wobei der Körper als flexibler, über seine gesamte Länge geschlitzter Kunststoffschlauch ausgebildet ist, der mit einer antibakterielle Wirkung aufweisenden Beschichtung versehen ist.

Die Gestaltung des Körpers als geschlitzter Schlauch ermöglicht es, diesen auf einfachste Art und Weise im Wundbereich einzusetzen, wobei die Flexibilität des Schlauches es gestattet, diesen hinsichtlich seiner Form an die jeweils vorgegebenen geometrischen Bedingungen im Bereich der Wunde anzupassen. Die Schlauchgestaltung gestattet es insbesondere, den Schlauch im Zusammenhang mit chirurgischen oder orthopädischen Instrumenten zu verwenden. Es wird als besonders vorteilhaft angesehen, wenn der flexible Schlauch aus Polyvinylchlorid (PVC) besteht. PVC-weich ist ein Werkstoff mit gummiähnlichen Eigenschaften.

Aufgrund der erfindungsgemäßen Gestaltung des Schlauches mit einem sich über seine gesamte Länge erstreckenden Schlitz ist es nicht erforderlich, diesen axial über ein orthopädisches Befestigungselement, beispielsweise einen orthopädischen Nagel oder eine orthopädische Schaube oder einen sonstigen Schlauch, zum Beispiel einen Drainageschlauch, zu schieben, sondern er kann durch Spreizung im Bereich seines Schlitzes radial auf das orthopädische Befestigungselement oder den von ihm zu umgebenden Schlauch aufgeschoben bzw. aufgesteckt werden. Der wesentliche Vorteil ist darin zu sehen, daß der antibakterielle Wirkung aufweisende Schlauch, sofern erforderlich, auf einfache Art und Weise ersetzt werden kann, indem er radial abgezogen und durch einen neuen Schlauch ersetzt wird. Es braucht damit keine eventuell vorgesehene externe Fixierung eines vom Schlauch umgebenen orthopädischen Befestigungselementes entfernt werden.

Es ist insbesondere daran gedacht, daß nur Schläuche bestimmer Länge bzw. Längenabstufung vorgehalten werden. Man kommt damit mit wenigen vorgegebenen Schlauchlängen aus. Diese, definierte Länge aufweisenden Schläuche sind zweckmäßig mit der antibakterielle Wirkung aufweisenden Beschichtung versehen, wobei die besondere Schlitzgestaltung des Schlauches es auf einfache Art und Weise-ermöglicht, diesen nicht nur außen, sondern auch innen und im Bereich seiner beiden Stirnseiten mit einer antibakteriell wirkenden Beschichtung zu versehen. Die Schlitzgestaltung des Schlauches ermöglicht es auch, diesen für unterschiedliche Durchmesser orthopädischer Befestigungselemente oder zu umgebender Schläuche zu verwenden, was die Anzahl vorzuhaltender Schlauchdurchmesser erheblich reduziert. So kann ein Schlauch sowohl für solche Befestigungselemente bzw. von diesem umgebene Schläuche Verwendung finden, deren Außendurchmesser dem Innendurchmesser des antibakterielle Wirkung aufweisenden Schlauches entspricht, mit der Konsequenz, daß der Schlauchschlitz geschlossen ist. Weist das orthopädische Befestigungselement bzw. der vom Schlauch umgebene Schlauch einen größeren Außendurchmesser auf, umschließt der die antibakterielle Wirkung aufweisende Schlauch diese Teile dennoch, allerdings ist dann ein geöffneter Schlitzspalt zu verzeichnen, das heißt es weitet sich der Schlauch um die Schlitzbreite auf.

Zweckmäßig ist der flexible Schlauch mit einer Silberbeschichtung versehen. Grundsätzlich kann der Körper auch mit jeder oligodynamischen Wirkung aufweisenden Beschichtung versehen sein, die diesen antibakteriellen Effekt auf Mikroorganismen hat. Es können hierdurch lokale Haut- und Gewebeinfektionen behandelt werden oder es findet der erfindungsgemäße Schlauch im Sinne einer präventiven Maßnahme unmittelbar nach dem Verwunden der Haut Verwendung.

Mit dem erfindungsgemäßen, antibakterielle Wirkung aufweisenden Schlauch können Leitungen zum Zuführen von Flüssigkeiten zum Körper oder zum Abführen unerwünschter Flüssigkeiten aus dem Körper versehen werden, insbesondere im Bereich des Eintritts des Schlauches in den Körper. Verwendung findet der Schlauch auch im Zusammenhang mit Bauteilen, die der Fixierung des Skelettes dienen. Der Schlauch hat die Fähigkeit diese Wundbereiche über eine lange Zeitspanne vor Mikroorganismen zu schützen oder bei vorhandenen Mikroorganismen zu behandeln.

Vorzugsweise wird der Schlauch in seiner Längsrichtung mittels eines auf-diesen und/oder einen Fixiernagel bzw. Fixierschraube geschobenen Ringelementes gehalten, das im Bereich des Austritts des Schlauches aus dem Körper des Patienten die Haut kontaktiert. Um eine Festlegung des Ringelements in Längsrichtung des Schlauches bzw. des Fixiernagels / der Fixierschraube zu gewährleisten, wird das Ringelement mit Preßsitz auf den Schlauch bzw. den Fixiernagel / die Fixierschraube geschoben. Abgesehen hiervon besteht die Möglichkeit, den Schlauch im Austrittsbereich aus dem Körper des Patienten mit der Haut zu vernähen. Sofern der Innendurchmesser des Schlauches geringer ist als der Außendurchmesser des Befestigungselementes oder des vom antibakterielle Wirkung aufweisenden Schlauch umgebenen Schlauches, kann gegebenenfalls auf ein Element zum Festlegen verzichtet werden, da wegen der Aufspreizung des Schlauches im Schlitzbereich der Schlauch sich auf dem von im umgebenen Teil verklemmt.

Weitere Merkmale der Erfindung sind in den Unteransprüchen, der Beschreibung der Figuren und den Figuren selbst dargestellt, wobei bemerkt wird, daß alle Einzelmerkmale und alle Kombinationen von Einzelmerkmalen weitere erfinderische Ausgestaltungen darstellen.

In den Figuren ist die Erfindung anhand mehrerer Ausführungsformen beispielsweise dargestellt, ohne auf diese Beschränkt zu sein.

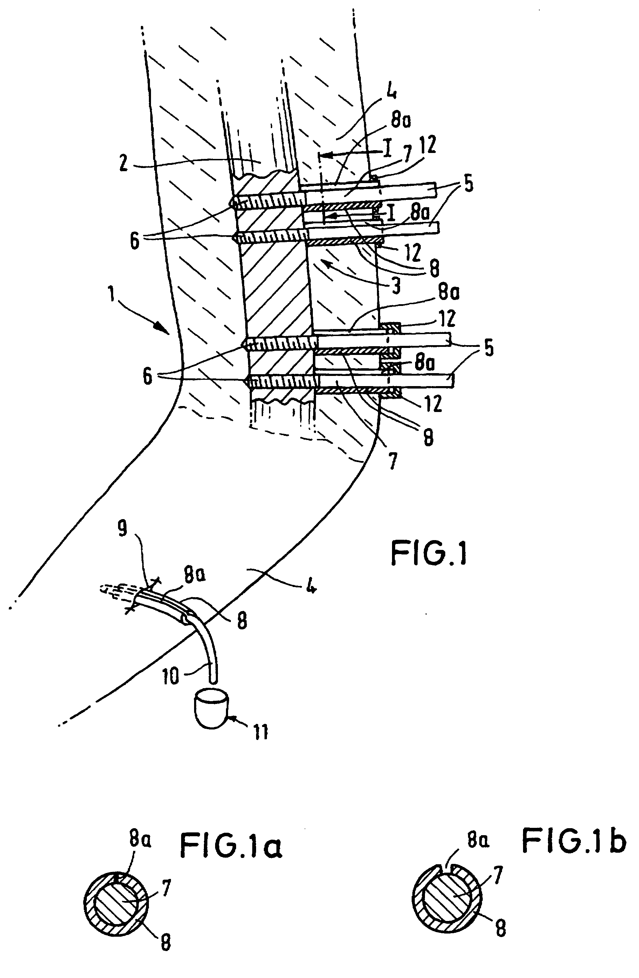

Figur 1 zeigt eine Extremität 1 eines menschlichen Körpers mit einem im Bereich eines Endes eines Knochens 2 angebrachten, nur teilweise dargestellten Fixateur externe 3. Dieser weist im dargestellten Bereich vier durch das Gewebe 4 der Extremität 1 durchgesteckte und in den Knochen 2 eingeschraubte Fixierschrauben 5 mit jeweils im dem Bereich des Knochens 2 befindlichem Gewindeabschnitt 6.sowie sonstigem, glattem Abschnitt 7 auf. Auf die metallischen Fixierschrauben 5 ist im Bereich des jeweiligen glatten Abschnittes 7 der antibakterielle Wirkung aufweisende, flexible Schlauch 8 aufgesteckt, der mit einem Ende am Knochen 2 anliegt und mit seinem anderen Ende außen aus dem Gewebe 4 herausragt, das heißt außen über die Haut der Extremität 1 übersteht. Zu den beiden oberen Fixierschrauben 5 ist die Ausführungsform eines als einfacher Ring ausgebildeten Ringelementes 12 aus Kunststoff gezeigt, das auf den flexiblen Schlauch 8 aufgeschoben ist und an der Haut des Patienten anliegt. Der Innendurchmesser des Ringelementes 12 ist dabei geringfügig geringer bemessen als der Außendurchmesser des Schlauches 8, so daß das Ringelement 12 aufgrund des Preßsitzes den Schlauch 8 axial fixiert, insbesondere indem der Schlauch 8 auf den glatten Abschnitt 7 der Fixierschraube 5 gedrückt wird. Die Fixierung des Schlauches 8 ließe sich beispielsweise auch durch eine aufgerauthe Ausgestaltung der Oberfläche von Schlauch 8 und/oder Ringelement 12 erreichen. Zu den beiden unteren Fixierschrauben 5 ist ein modizfiziertes Ringelement 12 gezeigt, das kappenartig ausgebildet ist. Das Ringelement 12 ist direkt auf den glatten Abschnitt 7 der Schraube 5 mit Preßsitz geschoben und mit dem axialen Ringabschnitt außen auf den Schlauch 8 aufgeschoben, wobei es die Haut des Patienten kontaktiert. Die erläuterten Ausführungsformen mit den Ringelementen finden bevorzugt Verwendung, wenn die Wunde nicht vernäht ist, sind aber auch bei den nachfolgend beschriebenen Anwendungsfällen einsetzbar.

Die Figur 1a zeigt einen Schnitt gemäß der Linie I-I in Figur 1 durch den Schlauch und den von diesem umgebenen glatten Abschnitt 7 der Fixierschraube 5. Figur 1a veranschaulicht, daß der Schlauch 8 mit einem Schlitz 8a versehen ist, der sich über die gesamte Länge des Schlauches 8a erstreckt. Der Innendurchmesser des Schlauches 8 ist vor dessen Aufstecken bzw. Aufschieben auf den Abschnitt 7 der Fixierschraube 5 gleich dem Außendurchmesser des glatten Abschnittes 7, so daß sich die freien Längskanten des Schlauches 8 kontaktieren, der Schlitz 8a somit geschlossen ist. Ein solcher Schlauch wäre durchaus auch für eine Fixierschraube 5 verwendbar, deren glatter Abschnitt 7 einen größeren Außendurchmesser aufweist. Dies ist in Figur 1b gezeigt. In der Konsequenz wird der Schlauch 8 durch den glatten Abschnitt 7 aufgespreizt, womit sich zwischen den beiden freien Längskanten des Schlauches 8 der geöffente Spalt 8a ergibt. In diesem Fall ist der Schlauch 8 auf dem glatten Abschnitt 7 geklemmt,-womit es keiner weiteren Fixierung des Schlauches 8 in vorbeschriebenem Sinne bedarf.

Die Ausführungen zur Detaildarstellung nach den Figuren 1a und 1b gelten sinngemäß für die nachfolgende Ausführung betreffend eine Abwandlung des erfindungsgemäßen Körpers.

Figur 1 zeigt für den unteren Bereich der Extremität 1 einen aus einer vernähten Wunde 9 herausragenden Drainageschlauch 10, der zu einem Auffangbehältnis 11 führt. Im Bereich der vernähten Wunde 9 umgibt der antibakterielle Wirkung aufweisende, flexible, geschlitzte Schlauch 8 den Drainageschlauch 10, das heißt, es ist der flexible Schlauch 8 in das Gewebe 4 eingesetzt ist und ragt außen teilweise aus diesem heraus und ist innen bis zum zentralen Wundgebiet geführt. Durch das Vernähen der Wunde wird der flexible, antibakterielle Wirkung aufweisende Schlauch 8 im Wundbereich gehalten.

Bei den Ausführungen des in Figur 1 gezeigten Schlauches 8 wird dieser damit als Hülle über existierende Vorrichtungen, die das Gewebe durchsetzen, gezogen, zum Beispiel über den gezeigten Drainageschlauch oder die externe Fixierschraube bzw. den Stift. Beim (erstmaligen) Anbringen der Vorrichtungen wird in aller Regel die Möglichkeit bestehen, den Schlauch 8 in seiner Axialrichtung auf das orthopädische Befestigungselement, den vom antibakterielle Wirkung aufweisenden Schlauch umgebenen Schlauch usw. aufzuschieben. Sind diese Vorrichtungen angebracht, ist es nicht ohne weiteres möglich, den antibakterielle Wirkung aufweisenden Schlauch zu entfernen, das heißt in seiner axialen Richtung abzuziehen und durch einen neuen zu ersetzen. Ein radiales Entfernen bzw. Aufbringen des Schlauches ist aber wegen des durchgehenden Schlitzes 8a des Schlauches 8 möglich.

Der flexible, antibakterielle Wirkung aufweisende Schlauch 8 besteht insbesondere aus PVC und ist bevorzugt über seine gesamte Oberfläche mit einer Silberbeschichtung versehen.

## Patentansprüche

1. Antibakterielle Wirkung aufweisender Körper zur Verwendung als medizinisches oder chirurgisches Hilfsmittel, wobei der Körper als flexibler Kunststoffschlauch (8), der mit einer antibakterielle Wirkung aufweisenden Beschichtung versehen ist, ausgebildet ist und der Schlauch (8) über seine gesamte Länge geschlitzt ist.

2. Körper nach Anspruch 1, wobei der Schlauch aus Polyvinylchlorid besteht.

3. Körper nach Anspruch 1 oder 2, wobei die die antibakterielle Wirkung aufweisende Beschichtung eine Silberbeschichtung ist.

4. Körper nach einem der Ansprüche 1 bis 3, wobei die gesamte Oberfläche des Schlauches (8) mit der Beschichtung versehen ist.

5. Körper nach einem der Ansprüche 1 bis 4, wobei der mit der antibakteriell wirkenden Beschichtung versehene Schlauch (8) zur Umgebung eines orthopädischen Befestigungselementes (5), insbesondere eines orthopädischen Nagels oder einer orthopädischen Schraube (5), angepaßt ist.

6. Körper nach einem der Ansprüche 1 bis 4, wobei der mit der antibakteriell wirkenden Beschichtung versehene Schlauch (8) zur Umgebung eines Schlauches (10), insbesondere eines Drainageschlauches, angepaßt ist.

7. Körper nach einem der Ansprüche 5 oder 6, wobei der Innendurchmesser des Schlauches (8) (vor dem Aufstecken bzw. Aufschieben) gleich oder geringer ist als der Außendurchmesser des orthopädischen Befestigungselementes (5) oder des Schlauches (10).

8. Körper nach einem der Ansprüche 1 bis 7, wobei der Schlauch (8) so angepaßt ist, daß er das Aufschieben eines für seine Fixierung dienenden Ringelementes (12) auf sich ermöglicht.

## Claims

1. Body possessing antibacterial action for use as medical or surgical aid, wherein the body is formed as flexible plastic tube (8) provided with a coating possessing antibacterial action and the tube is split over its entire length.

2. Body according to Claim 1 wherein the tube consists of polyvinyl chloride.

3. Body according to Claim 1 or Claim 2 wherein the coating possessing the antibacterial action is a silver coating.

4. Body according to any one of Claims 1 to 3 wherein the entire surface of the tube (8) is provided with the coating.

5. Body according to any one of Claims 1 to 4 wherein the tube (8) provided with the antibacterially active coating is adapted to surround an orthopaedic fastening element (5), in particular an orthopaedic nail or an orthopaedic screw (5).

6. Body according to any one of Claims 1 to 4 wherein the tube (8) provided with the antibacterially active coating is adapted to surround a tube (10), in particular a drainage tube.

7. Body according to Claim 5 or Claim 6 wherein the inner diameter of the tube (8) (before it is slipped on and/or pushed on) is equal to or smaller than the outer diameter of the orthopaedic fastening element (5) or tube (10).

8. Body according to any one of Claims 1 to 7 wherein the tube (8) is adapted so that a ring element (12) can be pushed on to it to secure it.

## Revendications

1. Corps présentant une activité antibactérienne à utiliser comme ustensile médical ou chirurgical, dans lequel le corps est réalisé sous la forme d'un tuyau flexible (8) en matière synthétique, qui est muni d'un recouvrement présentant une activité antibactérienne, et le tuyau (8) est muni d'une fente sur toute sa longueur.

2. Corps selon la revendication 1, dans lequel le tuyau est constitué de chlorure de polyvinyle.

3. Corps selon la revendication 1 ou 2, dans lequel le recouvrement présentant une activité antibactérienne est un recouvrement en argent.

4. Corps selon l'une quelconque des revendications 1 à 3, dans lequel la surface totale du tuyau (8) est munie du recouvrement.

5. Corps selon l'une quelconque des revendications 1 à 4, dans lequel le tuyau (8) muni du recouvrement à activité antibactérienne est conçu pour entourer un élément de fixation orthopédique (5), en particulier un clou orthopédique ou une vis orthopédique (5).

6. Corps selon l'une quelconque des revendications 1 à 4, dans lequel le tuyau (8) muni du recouvrement à activité antibactérienne est conçu pour entourer un tuyau (10), en particulier un tuyau de drainage.

7. Corps selon l'une quelconque des revendications 5 ou 6, dans lequel le diamètre interne du tuyau (8) (avant l'enfichage respectivement l'enfilage) est égal ou inférieur au diamètre externe de l'élément de fixation orthopédique (5) ou du tuyau (10).

8. Corps selon l'une quelconque des revendications 1 à 7, dans lequel le tuyau (8) est conçu de telle sorte qu'il permet d'enfiler sur lui-même un élément annulaire (12) pour sa fixation.
